(19) Europäisches Patentamt / European Patent Office / Office européen des brevets

(11) **EP 3 212 104 B1**

(12) **EUROPEAN PATENT SPECIFICATION**

(45) Date of publication and mention
of the grant of the patent:
**13.11.2019 Bulletin 2019/46**

(21) Application number: **15739611.0**

(22) Date of filing: **22.07.2015**

(51) Int Cl.:
**A61B 34/20** *(2016.01)*    *A61B 90/00 (2016.01)*

(86) International application number:
**PCT/EP2015/066786**

(87) International publication number:
**WO 2016/066287 (06.05.2016 Gazette 2016/18)**

(54) **HYBRID NAVIGATION SYSTEM FOR SURGICAL INTERVENTIONS**

HYBRIDES NAVIGATIONSSYSTEM FÜR CHIRURGISCHE EINGRIFFE

SYSTÈME DE NAVIGATION HYBRIDE POUR INTERVENTIONS CHIRURGICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.10.2014 GB 201419415**

(43) Date of publication of application:
**06.09.2017 Bulletin 2017/36**

(73) Proprietor: **Scopis GmbH
10179 Berlin (DE)**

(72) Inventors:
• **KOSMECKI, Bartosz**
  **10179 Berlin (DE)**
• **ÖZBEK, Christopher**
  **10823 Berlin (DE)**
• **REUTTER, Andreas**
  **10439 Berlin (DE)**
• **WINNE, Christian**
  **13127 Berlin (DE)**

(74) Representative: **Röthinger, Rainer et al
Wuesthoff & Wuesthoff
Patentanwälte PartG mbB
Schweigerstrasse 2
81541 München (DE)**

(56) References cited:
**WO-A2-01/34050        DE-A1-102007 054 450
US-A1- 2006 058 604**

**Description**

FIELD OF THE INVENTION

The present disclosure relates to a system for navigating surgical instruments in a patient and a method for navigating a surgical instrument in a patient.

BACKGROUND OF THE INVENTION

In surgical navigation, the position of used instruments such as pointer tools, suction tubes or endoscopes is visualized in the 3-D image data of the patient (e.g. CT, MRI). Different tracking technologies are known for determining the spatial position of instruments and of the patient.

Optical tracking systems usually comprise a camera system of two or more cameras in order to determine the pose (3D position and orientation) of optical trackers. These trackers can be rigid bodies of two or more optical markers (e.g. retro-reflective spheres or active light emitting diodes), which are attached to the instrument used and to the patient. In neurosurgery, one optical tracker is often rigidly fixed to a skull clamp, which is connected with the operating table and used to immobilize the head of the patient. Alternatively, the tracker can be fixed directly at the cranial bone with bone screws for instance.

As an alternative, electromagnetic tracking systems are known, which comprise a field generator and small sensor coils that can easily be integrated into surgical instruments. The field generator is positioned near the operating field using an articulated arm or can also be integrated into the operating table. The advantage of electromagnetic tracking is an easier handling in comparison to the optical tracking systems.

Optical systems suffer from the line-of-sight problem and the navigation camera must be manually positioned and aligned toward the operating field. A disadvantage of electromagnetic tracking is the susceptibility to errors in case of ferromagnetic material in the measurement region. This leads to distortions of the electromagnetic field, which can result in inaccurate position data.

In navigated microscopic neurosurgery, operating microscopes can also be integrated by attaching a tracker to the microscope to allow the measurement of its spatial position relative to the patient. This enables the navigation of the focal point of the microscope as well as the overlay of navigation information or data from a pre-operative surgical planning in the microscopic camera image. Therefore, a calibration of the projective properties of the microscopic camera system must be performed. Due to the possible changes of zoom and focus settings of the microscope during the surgical procedure, the camera properties need to be recorded for all microscope settings. This time-consuming calibration is typically done once during system installation and requires a repeatable tracker attachment at the microscope.

Due to the materials used and the built-in electric motors in particular, the risk is given that microscopes disturb the electromagnetic measurement field. Therefore, an accurate tracking of operating microscope with electromagnetic measurement systems is not a feasible task. Thus, there is a need for a system, which is easy and quick to handle, but provides reliable data about the exact position of used instruments.

Document WO 2001/034050 A2 (US 6,235,038 B1) discloses a system for utilizing and registering at least two surgical navigation systems during stereotactic surgery. The disclosed system comprises a first surgical navigation system defining a first patient space, a second surgical navigation system defining a second patient space, and a translation device to register the coordinates of the first patient space to the coordinates of the second patient space. The translation device comprises a rigid body, at least one component for a first navigation system placed in or on the rigid body, and at least one component for a second navigation system placed in or on the rigid body, in known relation to the at least one component for the first navigation system. The translation device is positioned in a working volume of each of the at least two navigation systems. The disclosed translation device as a rigid body manages only the registration between the coordinate systems associated with the tracking devices (patient spaces), a further linking to optical tracked microscopes or other instruments is not described. Furthermore, the translation device is inseparable, whereby the components of the first and second navigation system of the translation device cannot be separated and used independently.

Document US 2012/0165657 A1 describes a hybrid tracking system utilizing at least one combined light emitting diode (LED) and magnetoresistance sensor. The hybrid tracking system includes optical tracking technology and electromagnetic (EM) tracking technology with at least one combined LED and magnetoresistance reference sensor attached to a fixed object, at least one combined LED and magnetoresistance sensor attached to an object being tracked, and a processor coupled to the at least one combined LED and magnetoresistance reference sensor and the at least one combined LED and magnetoresistance sensor for processing signals from the at least one combined LED and magnetoresistance reference sensor and the at least one combined LED and magnetoresistance sensor. The characteristic of the described combined sensor lies in the integration of the LED and magnetoresistance sensor into one single unchangeable package. As a result of this integration, the components of the optical and electromagnetic tracking cannot be separated and used independently.

Documents US 20110054293 A1 and US 8494614 B2 discloses a navigation system or combination of navigation systems, which can be used to provide two or more navigation modalities to navigate a single instrument in a volume. For example, both an Electromagnetic (EM) and Electropotential (EP) navigation system can be used to navigate an instrument within the volume. Image data can also be illustrated relative to a tracked position of the instrument in the volume for navigation. This document describes only the registration of coordinate systems, which are associated with two or more tracking systems, which tracks one instrument simultaneously. The determined simultaneous tracking data are used to compute a registration to translate positions from one tracking system coordinate system to another.

US 7,702,379 B2 discloses a system and method for hybrid tracking in surgical navigation. The system according to the disclosure comprises the use of a plurality of tracking technologies in a medical procedure where a reconciler determines an active tracking technology. The reconciler determines the active tracking technology during the medical procedure. A switch may then activate one or more tracking technologies. The determination of which technology or technologies are to be activated may be based, for example, on metrics measured by each of the technologies, such as an accuracy measurement. In addition, a display may present representations based on at least data obtained by one or more of the tracking technologies. The switch may employ weighted switching to gradually switch the display of a first representation corresponding to a first tracking technology to the display of a second representation corresponding to a second tracking technology, where the first technology is deactivated and the second technology is activated. This document describes only a method for selecting the active tracking system which is used for navigation from a plurality of available tracking systems based on metrics which are associated with each tracking system. The disclosed method does not combine the tracking data of different tracking systems.

The International application WO 2000039576 A1 discloses a system that tracks the 3-dimensional position and orientation of one or more bodies in a volume by a light based, as well as at least one non-light based mensuration sub-system. Such a system overcomes the limitation of light based mensuration systems to the necessity of the bodies to be in constant line-of-sight of its light based position sensors. According to the disclosure, the system possesses most of the accuracy and stability of its light based position measurement sub-system, but can also work without direct line of sight either for short periods of time or within certain parts of the volume. The system incorporates other sensors, such as inertial or magnetic sensors, which are frequently recalibrated against the light based sub-system while the bodies are visible by the light based sub-system. The disclosure is limited to a system that is only a tool equipped with localizers for two tracking systems (optical and EM).

In US 5831260 A hybrid motion tracker is disclosed, which captures the motion of a person. Magnetic field sensors and optical sources are placed on a person, each located on different limbs. A fixed transmitter emits electromagnetic energy and infrared light is transmitted from the optical light sources to the fixed optical sensors. The magnetic field sensors sense the magnetic field and a computer calculates each sensor's position and orientation relative to the fixed transmitter. The optical system's Position Sensing Detectors measure the transmitted infrared light and the computer calculates the position and orientation of each optical light source. The position and orientation of each sensor is used to reconstruct the person's motion in real time, which is sent to a host computer. The computer utilizes the optical system which is more precise than the magnetic field system to compensate for the magnetic field system and thereby achieve higher accuracies. The system is a hybrid system using both magnetic fields and infrared light. By combining the two different technologies into one system, accuracy and dynamic performance are enhanced over results obtained through sole use of a magnetically based system. The system refers only to a hybrid tracker for motion tracking of human persons. There is neither an application for surgical navigation with microscopy nor the registration to preoperative image data disclosed or proposed.

In summary, the existing systems and methods from the state of the art combine at least two different tracking systems in order to improve the availability or accuracy of the tracking data for single instruments.

BRIEF SUMMARY OF THE INVENTION

The invention is defined in the appended claims. The present disclosure provides a system for navigating surgical instruments relative to a patient, comprising the following components:

- a first tracking system for measuring the position of at least one of a first type of tracker;
- a second tracking system for measuring the position of at least one of a second type of tracker;
- at least one patient tracker belonging to the first type of tracker measuring the position of the patient;
- a surgical instrument comprising a tracker belonging to the second type of tracker and at least one geometric primitive of interest; and
- a connector for rigidly, but removably connecting the at least one of a first type of tracker with at least one of a second type of tracker for registration and/or transformation of the coordinates of the surgical instrument to the coordinates of the image data of the patient and vice versa wherein the connector allows that the separate trackers can be removably connected by the user during preparation of system and even during surgery.

The first tracking system can be an electromagnetic tracking system and the second tracking system can be an optical tracking system.

The system can further comprise a hybrid navigation unit comprising at least a computer system for providing simultaneous support for the first and second tracking system.

It is intended that the at least one of a first type of tracker or the at least one of a second type of tracker may be connected to the hybrid navigation unit.

The connector of a system of the present invention can be a hybrid tracker comprising stored information of pre-operatively known transformation between the trackers.

It is further envisaged that the at least one patient tracker which belongs to the at least one of a first type of tracker and at least one patient tracker which belongs to the at least one of a second type of tracker can be rigidly, but removably fixed to each other via the connector at the beginning or during surgery. The poses of the trackers relative to each other may be known from construction data.

It is further envisaged that the at least one patient tracker which belongs to the at least one of a first type of tracker and at least one patient tracker which belongs to the at least one of a second type of tracker can be rigidly fixed to the patient.

A connector not in accordance with the present invention may further consist of a solid, hard tissue of the patient for connecting the two or more patient trackers of both types of trackers, being rigidly fixed relative to each other and to the operating region of interest.

The surgical instrument of a system of the present disclosure can be an electrically powered and its geometric primitive of interest is the tip of the surgical instrument.

The surgical instrument can also be an operating microscope and the geometric primitives of interest are at least one of the image rectangle in the focal plane, the focus point or the camera origin of the operating microscope.

The system of the present disclosure may further comprise a monitor for displaying the real microscopic image superimposed with a virtual microscopic image or an image injection module in the microscope for displaying a virtual microscopic image for visual perception of the surgeon when looking through the oculars.

Another object of the present disclosure is a method for navigating a surgical instrument relative to a patient, the method comprising the steps of

- connecting at least one of a first type of tracker with at least one of a second type of tracker by using a connector;
- measuring the position of at least one of a first type of tracker with a first tracking system;
- measuring the position of a surgical instrument by using at least one of a second type of tracker with a second tracking system;
- measuring the position of the patient with at least one patient tracker belonging to the at least one of a first type of tracker;
- registering and/or transforming the coordinates of the surgical instrument and its geometric primitive of interest to the coordinates of the image data of the patient and vice versa.

The method may further comprise the step of using an electromagnetic tracking system as first tracking system and an optical tracking system as second tracking system.

It is further intended that the method may comprise the step of using a device comprising stored information of pre-operatively known transformation between the trackers for registering and/or transforming the coordinates of a surgical instrument and its geometric primitive of interest to the coordinates of the image data of the patient and vice versa.

Furthermore it is intended that the step of connecting at least one of a first type of tracker with at least one of a second type of tracker by using a connector may be conducted pre- or intra-operatively by the user so that the trackers are then rigidly fixed relative to each other in a known or approximately known spatial relation. Furthermore the method may comprise the step of verifying and/or optimizing a previously known or approximately known transformation between these trackers. The trackers may be rigidly, but removably fixed to the connector.

It is further intended that the step of connecting at least one of a first type of tracker with at least one of a second type of tracker by using a connector is conducted with two patient trackers, which are then rigidly, but removably fixed relative to each other in a known or approximately known spatial relation. Furthermore the method may comprise the step of verifying and/or optimizing a previously known or approximately known transformation between these patient trackers.

Furthermore the method may comprise the step of connecting intra-operatively two or more patient trackers of both types of trackers, which are rigidly fixed relative to each other, and the step of intra-operatively determining the transformation between these trackers.

The transformation between the patient trackers may be determined, optimized or verified by performing two patient image registration procedures, one for the patient trackers of the at least one of a first type of tracker und one for the patient trackers of the at least one of a second type of tracker.

Alternatively or in addition the transformation between the patient trackers may be determined, optimized or verified by performing one patient image registration procedures using a navigated instrument equipped with trackers of both types of tracker.

It is further intended that the transformation between the trackers or patient trackers may be determined, optimized or verified by sampling features whose spatial positions are known relative to a tracker or patient tracker of one type of tracker using a navigated instrument equipped with a tracker of the other type of tracker. These features can include touch points, plane surfaces or faces of geometric primitives which may be a part of a rigid body of the patient tracker of at least one type of tracker.

An electrically powered tool may be used as surgical instrument or an operating microscope can be as surgical instrument.

The coordinates of the image data of the patient may be transformed into the reference coordinate system of the microscopic camera enabling the generation of a virtual microscopic image.

Further, the virtual microscopic image can be superimposed on the real microscopic image on a monitor of the navigation system.

The virtual microscopic image may further be injected into the microscope beam path for visual perception of the surgeon when looking through oculars.

A further object of the present disclosure is the use of a system for navigating surgical instruments relative to a patient, comprising a first tracking system for measuring the position of at least one of a first type of tracker, a second tracking system for measuring the position of at least one of a second type of tracker, at least one patient tracker belonging to the first type of tracker measuring the position of the patient, a surgical instrument with comprising a tracker belonging to the second type of tracker; and a connector for rigidly connecting the at least one of a first type of tracker with at least one of a second type of tracker for registration and/or transformation of the coordinates of the surgical instrument to coordinates of the image data of the patient and vice versa in surgical interventions

BRIEF DESCRIPTION OF THE FIGURES

The present disclosure will be described by figures and examples. It is obvious for a person ordinary skilled in the art that the scope of the disclosure is not limited to the disclosed embodiments. It shows:

Figure 1    Hybrid navigation system for microscopic neurosurgery using a hybrid tracker

Figure 2    Reference coordinate systems of the first embodiment

Figure 3    Second embodiment of the disclosure using an optical and an electromagnetic patient tracker for patient tracking

Figure 4    Reference coordinate systems of the second embodiment when using a direct registration of the patient trackers after the fixation to the patient

DETAILED DESCRIPTION OF THE INVENTION

The objective of the disclosure is the integration of microscopic imaging into an electromagnetic navigation system. The advantages of the electromagnetic tracking with the simplified handling of instruments, the unrestricted visibility and easier mounting of the patient tracker are retained and expanded to include a uncomplicated integration of microscopy.

The present disclosure provides a hybrid navigation system that combines two or more tracking systems and may comprise the following functional groups:

- Hybrid navigation unit with simultaneous support for two or more tracking systems
- First tracking system for measuring the pose of trackers of a first tracker type
- Second tracking system for measuring the pose of trackers of a second tracker type
- Tracker of the first tracker type for spatial position measurement of the patient
- Rigid assembly of two or more trackers of either the first or the second tracker type using a connector
- Navigated pointer instrument with an attached or integrated tracker of the first tracker type for patient image registration
- Surgical instrument with attached or integrated tracker of the second tracker type with a geometric primitive of interest (e.g. operating microscope or active instruments like drills or shavers)

The basic part of the system is the hybrid navigation unit, which may comprise a computer system and electronic parts of the two tracking systems. Other components of the tracking systems can be hooked up to the navigation unit in order to establish a communication for reporting measurement results or for direct connecting of tracking components to integrated parts of the tracking systems. In case of using an optical tracking system, a navigation camera can be connected. In case of using an electromagnetic tracking system, the field generator and the wires of the sensor coils may be connected to the hybrid navigation unit.

The computer system of the hybrid navigation unit processes the position and orientation data of the tracking systems and calculates 2-D and 3-D visualisations of the current position of used navigated instruments in the 3-D image data of the patient. These image data are imported from CD-ROM, USB storage device or PACS before surgery.

The further system components and their functions within the hybrid navigation systems are explained in the following descriptions of possible embodiments of this disclosure. It is obvious for a person ordinary skilled in the art that the scope of the disclosure is not limited to the disclosed embodiments.

This first embodiment of the disclosure is a hybrid navigation system, which combines electromagnetic pointer navigation with optical tracking of an operating microscope using a hybrid tracker.

In this embodiment, the first tracking system is an electromagnetic tracking system which comprises of a field generator which is a set of coils which generate an electromagnetic field in the operating area. The pose measurement of the electromagnetic tracking system is performed with the help of small sensor coils which are incorporated into electromagnetic trackers and the navigated electromagnetic instruments (e.g. pointer instruments, suctions). The electromagnetic tracking system measures the spatial position at least of the patient and of one instrument (e.g. a pointer instrument) which can be used for performing the registration procedure of the image data of the patient. The position of the patient is measured using a patient tracker which is rigidly attached to the operating field at the patient.

To enable the calculation of the position of instruments in the 3-D image data, a registration of the 3-D image data must be performed at the beginning of the navigated surgery. In this case, a transformation between the reference coordinate system of the 3-D image data and the reference coordinate system of the patient tracker is determined. In the literature several methods for registration of the patient image data are known like the landmark registration and the surface registration (Eggers et al., 2006, Int. J. Oral and Max. Surg., 35(12), 1081-1095; Maintz and Viergever, 1998, Med. Int. Ana. 2(1), 1-36.). Here, anatomical or artificial landmarks or surfaces marked in or derived from 3-D image data of the patient and are touched or captured with the navigated instruments (e.g. pointer instruments) at the patient.

The second tracking system in this embodiment is an optical tracking system, which measures the pose of optical trackers which are attached at rigid instruments. Optical trackers are rigid bodies equipped with usually three or more optical markers in a known spatial configuration which allows the measurement of the pose the tracker with six degrees of freedom. For measuring the pose of the microscope during surgery, the microscope is also equipped with an optical tracker which is rigidly and possibly reproducibly attached to the microscope.

The rigid, but removable assembly of two trackers of the two tracking systems may be realized in form of a hybrid tracker comprising a connector to removably connect a rigid body with at least three attached markers for optical tracking and sensor coils for electromagnetic tracking.

The hybrid tracker is used to provide a link between the reference coordinate systems associated with the electromagnetic and optical tracking system, respectively. The spatial relationship of the optical and the electromagnetic trackers and their defined reference coordinate systems has to be known. A possible embodiment of implementation may comprise the one-time calibration of the hybrid trackers during the manufacturing process. The transformation between the reference coordinate systems of the optical and electromagnetic tracker can be stored on a memory module of the electromagnetic tracker. During the intraoperative use, the hybrid navigation unit can read out and utilize the stored transformation data.

For the management and calculation of the spatial relationships between the various system components, reference coordinate systems are associated with these components. The following reference coordinate systems are defined:

- *img* for the image data of the patient

- *cam* for the navigation camera

- *fg* for the field generator

- *pt_em* for the electromagnetic patient tracker

- *hybrid_opt* for the optical tracker of the hybrid tracker

- *hybrid_em* for the electromagnetic tracker of the hybrid tracker

- *tool_opt* for the tracker of the optically tracked intrument

- *tool_em* for the electromagnetic tracked instrument

A transformation matrix for converting coordinates between one reference coordinate system ref1 and a second reference coordinate system ref2 is suitable to describe the relative pose of two reference coordinate systems. This transformation matrix $^{ref2}T_{ref1}$ can be used in the mapping rule $^{ref2}p = {}^{ref2}T_{ref1} * {}^{ref1}p$, which describes the computation of the homogeneous coordinates $^{ref2}p$ in the reference coordinate system *ref2* based on homogeneous coordinates $^{ref2}p$ in the reference coordinate system *ref2*.

The relevant transformations between the reference coordinate systems of the first embodiment are:

$^{cam}T_{tool\_opt}$: Rigid transformation between the reference coordinate system of the optical tracking system and the reference coordinate system of the optical tracker of the microscope. This transformation is continuously reported by the optical tracking system.

$^{cam}T_{hybrid\_opt}$: Rigid transformation between the reference coordinate system of the optical tracking system and the reference coordinate system of the optical tracker of the hybrid tracker. This transformation is continuously reported by the optical tracking system.

$^{video}T_{tool\_opt}$: Rigid transformation between the reference coordinate system of the optical tracker of the microscope and the reference coordinate system origin of the camera of the microscope according to the pin-hole camera model. This transformation is determined during a microscope calibration procedure for different microscope zoom und focus settings.

$^{hybrid\_em}T_{hybrid\_opt}$: Rigid transformation between the reference coordinate system of the electromagnetic tracker of the hybrid tracker and the reference coordinate system of the optical tracker of the hybrid tracker. This transformation is usually determined during manufacturing process and can be stored on a memory module in the electromagnetic tracker.

$^{fg}T_{hybrid\_em}$: Rigid transformation between the reference coordinate system of the electromagnetic tracking system and the reference coordinate system of the electromagnetic tracker of the hybrid tracker. This transformation is continuously reported by the electromagnetic tracking system.

$^{fg}T_{tool\_em}$: Rigid transformation between the reference coordinate system of the electromagnetic tracking system and the reference coordinate system of the electromagnetic tracker of the pointer instrument. This transformation is continuously reported by the electromagnetic tracking system.

$^{fg}T_{pt\_em}$: Rigid transformation between the reference coordinate system of the electromagnetic tracking system and the reference coordinate system of the electromagnetic patient tracker. This transformation is continuously reported by the electromagnetic tracking system.

$^{pt\_em}T_{img}$: Rigid transformation between the reference coordinate system of the electromagnetic patient tracker and the reference coordinate system of the image data of the patient. This transformation is determined during the patient image registration procedure at the beginning of the navigated surgery.

Using the presented transformations based on the defined reference coordinate systems, a transformation chain can be formed in order to easily transfer coordinates of a pre-operative planning within the image data of the patient $^{img}p$ into the microscope camera reference coordinate system "video":

$$^{video}p = {}^{video}T_{tool\_opt} * \left({}^{cam}T_{tool\_opt}\right)^{-1} * {}^{cam}T_{hybrid\_opt} * \left({}^{hybrid\_em}T_{hybrid\_opt}\right)^{-1} * \left({}^{hybrid\_em}T_{hybrid\_opt}\right)^{-1} * \left({}^{fg}T_{hybrid\_em}\right)^{-1} * {}^{fg}T_{pt\_em} * {}^{pt\_em}T_{img} * {}^{img}p$$

If the intrinsic camera properties (e.g. focal length f, image center $C_x$, $C_y$, aspect ratio $s_x$) of the microscope are also known from a previous calibration, the coordinates in the "video" reference coordinate system can be projected onto the image plane of the microscope according to the pin-hole camera model and used to generate virtual microscopic images containing preoperative planning data. An augmented reality visualization can then be realized by superimposing the real with the virtual microscopic image data. One way of implementation is the monitor-based augmented reality, in which the real image and the virtual image are superimposed on the screen. Alternatively, the option of injecting external image data into the optical channel of some microscopes allows a display of the virtual microscope image data directly in the field of view of the surgeon.

Furthermore, coordinates given in the reference coordinate system of the microscope tracker "tool_opt" (e.g. the position of the focal point of the microscope or the position of the microscope camera origin) have to be transformed into the reference coordinate system of the image data of the patient in order to visualize this position in the 3D or slice views of the image data. The computation of a coordinate in the reference coordinate system of the image data $^{img}p$ can also be done using the defined transformations:

$$^{img}\mathbf{p} = \left(^{pt\_em}\mathbf{T}_{img}\right)^{-1} * \left(^{fg}\mathbf{T}_{pt\_em}\right)^{-1} * {}^{fg}\mathbf{T}_{hybrid\_em} * {}^{hybrid\_em}\mathbf{T}_{hybrid\_opt} * \left(^{cam}\mathbf{T}_{hybrid\_opt}\right)^{-1} *$$
$$^{cam}\mathbf{T}_{tool\_opt} * {}^{tool\_opt}\mathbf{p}$$

The geometric primitive of interest of the microscope can be the focal point of the microscope, the position of the microscope camera origin according to the pin-hole camera model or the image rectangle in the focal plane of the microscope. For surgical instruments like drills or shavers, the position and shape of the instrument tip is the geometric primitive of interest. The shape of the geometric primitive of interest is usually modelled as a point, a sphere, a cylinder or a capsule.

The second embodiment of the disclosure is a hybrid navigation system, which combines electromagnetic pointer navigation with optical tracking of an operating microscope using an electromagnetic patient tracker and an optical patient tracker simultaneously.

The second embodiment also uses electromagnetic pointer tracking with an integration of an optical tracked operating microscope. The following system components are similar to the first embodiment: the hybrid navigation unit, the electromagnetic tracking system with the field generator, the optical tracking system with the navigation camera, the electromagnetic tracked pointer instrument for patient image registration and the operating microscope with an optical tracker.

The difference in comparison to the first embodiment is the usage of two patient trackers, where both are tracked with the corresponding tracking system, instead of using one patient tracker and a hybrid tracker. This means that the implementation of the rigid assembly of tracker elements of the first and the second tracking system is realized by a rigid attachment of the two patients tracker relative to the operating field. In analogy to the hybrid tracker, this results in a temporary rigid connection between one tracker of the electromagnetic and one tracker of the optical tracking system.

The spatial relationship between these two patient trackers is initially not know after the tracker fixation at the patient. Several methods are conceivable to determine the rigid transformation between the reference coordinate systems associated with these two trackers:

- *pt_em* for the electromagnetic patient tracker

- *pt_opt* for the optical patient tracker

One option is to perform two patient image registrations, one using electromagnetic and one using optical tracking. As a result, the transformations between the reference coordinate system of the image data of the patient and the reference coordinate system of the optical tracker $^{pt\_opt}\mathbf{T}_{img}$ and of the electromagnetic tracker $^{pt\_em}\mathbf{T}_{img}$ are known. This allows the computation of the rigid transform between the reference coordinate systems of the two patient trackers:

$$^{pt\_em}\mathbf{T}_{pt\_opt} = {}^{pt\_em}\mathbf{T}_{img} * \left(^{pt\_opt}\mathbf{T}_{img}\right)^{-1}$$

A second option is the use of a pointer tool equipped with an optical as well as an electromagnetic tracker. That allows the computation of the two patient image registrations for the optical and the electromagnetic patient tracker in one stage of registration procedure. The computation of the transformation $^{pt\_em}\mathbf{T}_{pt\_opt}$ is performed as shown in the description of the first option.

A third option is to perform only one patient image registration, for example using the electromagnetic pointer instrument in order to determine the transformation between the image data of the patient and the electromagnetic patient tracker. Then, given features of the other (optical) patient tracker (e.g. 3D points or planes) are sampled with the electromagnetic pointer and thus recorded in the reference coordinate system of the electromagnetic patients tracker. By knowing the location of these features in the reference coordinate system of the optical tracker, the rigid transformation between the reference coordinate systems of the optical and electromagnetic patient tracker $^{pt\_em}\mathbf{T}_{pt\_opt}$ can be calculated.

In addition to the described transformations of the first embodiment, in this second embodiment the following transformations are relevant:

$^{cam}\mathbf{T}_{pt\_opt}$: Rigid transformation between the reference coordinate system of the optical tracking system and the reference coordinate system of the optical patient tracker. This transformation is continuously reported by the optical tracking system.

$^{pt\_em}\mathbf{T}_{pt\_opt}$: Rigid transformation between the reference coordinate system of the electromagnetic patient tracker and the reference coordinate system of the optical patient tracker. This transformation can be determined at the beginning of the surgery for example using one of the three options which are described above.

With the transformation chain based on the defined reference coordinate systems, coordinates of a preoperative planning given in the reference coordinate system of the image data of the patient $^{img}\mathbf{p}$ can easily transformed into the reference coordinate system of microscope camera "video":

$$^{video}\mathbf{p} = \,^{video}\mathbf{T}_{tool\_opt} * (^{cam}\mathbf{T}_{tool\_opt})^{-1} * \,^{cam}\mathbf{T}_{pt\_opt} * (^{pt\_em}\mathbf{T}_{pt\_opt})^{-1} * \,^{pt\_em}\mathbf{T}_{img} * \,^{img}\mathbf{p}$$

If the intrinsic camera properties (e.g. focal length f, image center $C_x$, $C_y$, aspect ratio $s_x$) of the microscope are also known from a previous calibration, the coordinates in the "video" reference coordinate system can be projected onto the image plane of the microscope according to the pin-hole camera model and used to generate virtual microscopic images containing preoperative planning data. An augmented reality visualization can then be realized by superimposing the real with the virtual microscopic image data.

Furthermore, coordinates given in the reference coordinate system of the microscope tracker "tool_opt" (e.g. the position of the focal point or the position of the microscope camera origin) have be transformed into the reference coordinate system of the image data of the patient in order to visualize this position in the 3D or slice views of the image data. The computation of a coordinate in the reference coordinate system of the image data $^{img}p$ can also be done using the defined transformations:

$$^{img}\mathbf{p} = (^{pt\_em}\mathbf{T}_{img})^{-1} * \,^{pt\_em}\mathbf{T}_{pt\_opt} * (^{cam}\mathbf{T}_{pt\_opt})^{-1} * \,^{cam}\mathbf{T}_{tool\_opt} * (^{video}\mathbf{T}_{tool\_opt})^{-1} * \,^{video}\mathbf{p}$$

In addition, the computation and visualisation of navigation information may be beneficial, for example the visualisation of the expected position of the tip of the electromagnetic pointer instrument in the microscopic camera image in order to allow the surgeon to estimate the overlay error of navigation or planning data in the microscopic camera image:

$$^{video}\mathbf{p} = \,^{video}\mathbf{T}_{tool\_opt} * (^{cam}\mathbf{T}_{tool\_opt})^{-1} * \,^{cam}\mathbf{T}_{pt\_opt} * (^{pt\_em}\mathbf{T}_{pt\_opt})^{-1} * (^{fg}\mathbf{T}_{pt\_em})^{-1} * \,^{fg}\mathbf{T}_{tool\_em} * (0 \quad 0 \quad 0 \quad 1)^T$$

As a simplification of this system concept, a use of a hybrid patient tracker is also conceivable. In this case, the hybrid tracker, a fixed spatial arrangement of an electromagnetic tracker and an optical tracker, is rigidly attached directly to the patient. Thereby, the positional relationship between the optical and electromagnetic trackers $^{pt\_em}\mathbf{T}_{pt\_opt}$ is known.

The disclosure is new, because the combination of two or more tracking systems to combine electromagnetic navigation with microscopy has not been disclosed in the relevant state of the art. By using a rigid assembly of trackers of the two or more tracking systems, a registration of these tracking systems is realized in order to enable the tracking of the microscope with a second tracking device and combine the tracking information with the electromagnetic navigation data. Different intra-operative realisations of the rigid assembly of trackers are disclosed in order to enable the transformation of coordinates between the reference coordinate systems of the microscope, the image data and electromagnetic tracked instruments. This allows for the first time a feasible integration of microscopy to electromagnetic navigation.

The major advantage of the disclosure is the integration of electromagnetic navigation into surgical microscopes. The advantages of the realisation with a so-called hybrid tracker in form of a rigid but removable connection of at least two trackers of at least two types of trackers are:

- Easy placement of the hybrid tracker in the operating environment within the measurement fields of the used tracking systems. There is no need for a special fixation relative to the patient or a tracking device.
- Removably connected tracker components may also be used separately for pose measurement of the patient or of surgical instruments if they are not connected.
- There are no additional requirements to the fixation of the electromagnetic patient tracker at the patient. In particular, the electromagnetic tracker can be tucked under the sterile covering of the patient.
- In addition to the registration of the patient image registration, no further intraoperative calibration is needed.
- The patient may be located outside the measurement field of the second tracking system. Only the hybrid tracker must be positioned measurement fields of the used tracking systems. In case of an optical tracking system, this can significantly reduce line-of-sight problems during surgery.

The advantages of the second embodiment disclosed in the instant disclosure is an increased system accuracy, because less trackers are used so that the influence of the inaccuracies of the pose measurement might be lower.

DETAILLED DESCRIPTION OF THE FIGURES

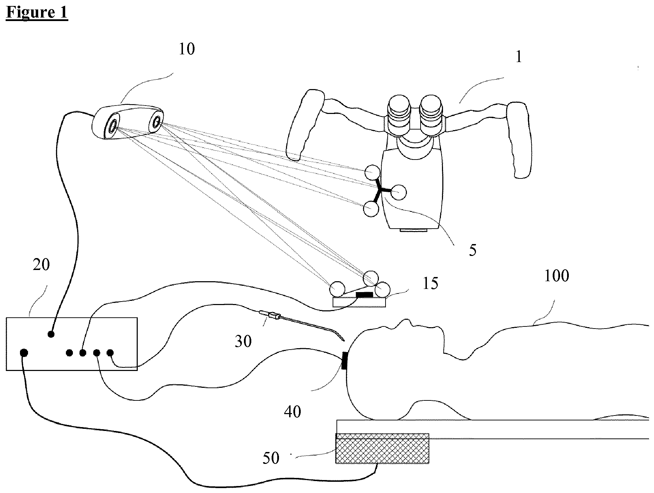

Figure 1 discloses a first embodiment of the hybrid navigation system, which combines electromagnetic pointer navigation with optical tracking of an operating microscope 1 using a hybrid tracker 15. The figure 1 shows the system components and their tracking connections. The field generator 50 of the electromagnetic is attached to the operating table 90 and is connected to the hybrid navigation unit 20. Additionally, the electromagnetic trackers and electromagnetic tracked instruments as well as the navigation camera 10 as a part of the optical tracking system are connected to the hybrid navigation unit 20. The patient tracker is rigidly fixed to the operating field of the patient 100. In neurosurgery, the patient tracker is possibly attached to the skull bone of the patient 100. The electromagnetic tracked pointer instrument 31 is used for patient registration of the 3D image data of the patient 100 and for usual pointer navigation. The hybrid tracker 15 must be positioned within the measurement field of the electromagnetic and the optical tracking system so that the pose of the optical and electromagnetic tracker of the hybrid tracker 15 are simultaneously measured by the two tracking systems. In addition, the navigation camera 10 tracks the pose of the microscope with the help of the microscope tracker 5, which is rigidly attached to the operating microscope 1. The visual position measurement is illustrated by the lines of sight between the navigation camera 10 and the markers of the optical trackers.

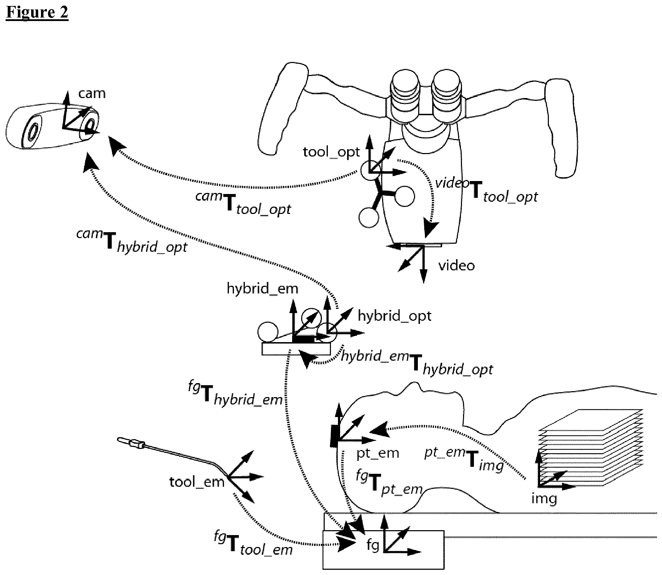

Figure 2 illustrates the reference coordinate systems associated with the system components of the first embodiment. Additionally, relevant transformations between the reference coordinate systems are visualised using dashed arrows. The visualised transformations are either measured with one of the tracking systems or determined during a registration or calibration process. The measurement results of the electromagnetic tracking system are continuously made available in form of the transformations $^{fg}\mathbf{T}_{hybrid\_em}$, $^{fg}\mathbf{T}_{tool\_em}$ and $^{fg}\mathbf{T}_{pt\_em}$. The measurement results of the optical tracking system are continuously made available in form of the transformations $^{cam}\mathbf{T}_{hybrid\_opt}$ and $^{cam}\mathbf{T}_{tool\_opt}$. The transformation $^{video}\mathbf{T}_{tool\_opt}$ is usually determined in a calibration procedure during system installation and is depending on the current zoom und focus settings of the microscope. The transformation $^{hybrid\_em}\mathbf{T}_{hybrid\_opt}$ describes the spatial relationship between the electromagnetic and optical trackers of the hybrid tracker 15. The transformation $^{pt\_em}\mathbf{T}_{img}$ stores the result of the image data registration which is determined at the beginning of the navigated surgery.

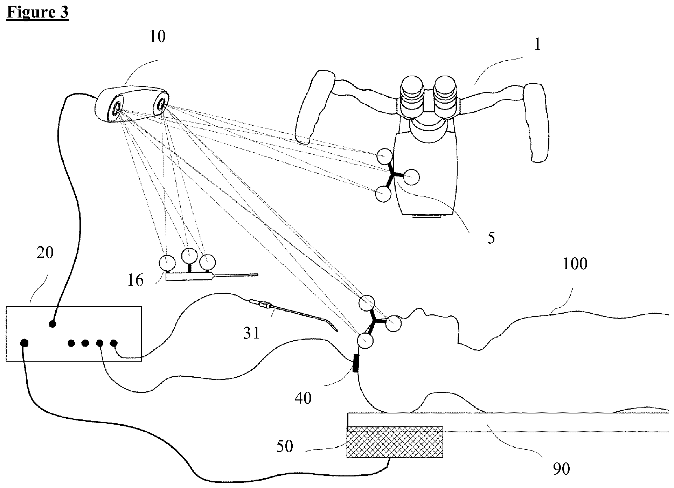

Figure 3 discloses a second embodiment of the hybrid navigation system which combines electromagnetic pointer navigation with optical tracking of an operating microscope 1 using an optical and an electromagnetic patient tracker 40. These both patient trackers 40 are rigidly attached to the operating field of the patient 100 at the beginning of the surgery. As the trackers are attached rigidly to the patient 100, they result in a rigid assembly of two trackers of the two different tracking systems. The spatial relationship between the two patient trackers 40 must be determined intra-operatively. For that, the registrations of the trackers to a common reference coordinate system (e.g. of the patient 3D image data) can be used. Alternatively the reference coordinate systems of the two trackers are registered directly by sampling feature elements, which are known in the reference coordinate system of one tracker with a tracked instrument associated with the other tracking system.

The illustrated system components hybrid navigation unit 20, the navigation camera 10, the field generator 50, the electromagnetic tracked pointer instrument 31 and the operating microscope 1 with its optical tracker are similar to the first embodiment.

The shown optical tracked pointer instrument 16 can be used for registration of the patient image data relative to the optical patient tracker 40. If direct registration of the patient trackers 40 using feature elements at the optical tracker is performed, the optical tracked pointer instrument 16 is not necessary.

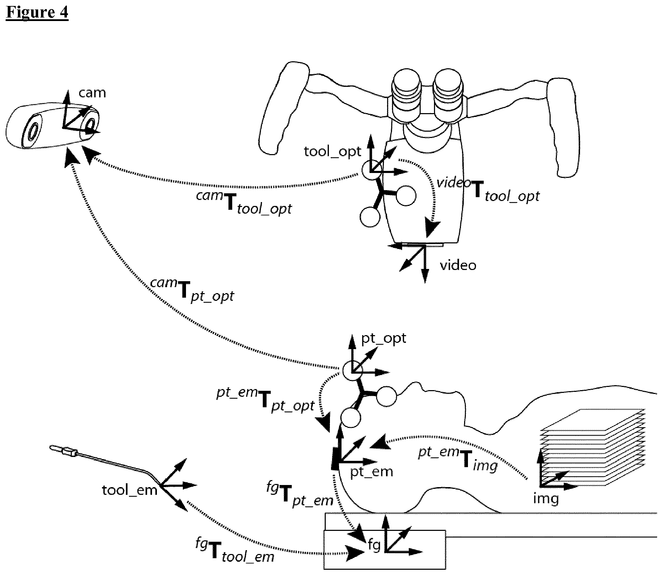

Figure 4 illustrates the reference coordinate systems associated with the system components of the second embodiment using direct registration of the two patient trackers 40. Additionally, relevant transformations between the reference coordinate systems are visualised using dashed arrows. The visualised transformations are either measured with one of the tracking systems or determined during a registration or calibration process. The measurement results of the electromagnetic tracking system are continuously made available in form of the transformations $^{fg}\mathbf{T}_{pt\_em}$ and $^{fg}\mathbf{T}_{tool\_em}$. The measurement results of the optical tracking system are continuously made available in form of the transformations $^{cam}\mathbf{T}_{pt\_opt}$ and $^{cam}\mathbf{T}_{tool\_opt}$. The transformation $^{video}\mathbf{T}_{tool\_opt}$ is usually determined in a calibration procedure during system installation and is depending on the current zoom und focus settings of the microscope. The transformation $^{pt\_em}\mathbf{T}_{pt\_opt}$ describes the spatial relationship between the electromagnetic and optical trackers, which is determined intra-operatively by sampling feature points on the optical patient tracker 40 using the electromagnetic pointer instrument 31. The transformation $^{pt\_em}\mathbf{T}_{img}$ stores the result of the image data registration which is determined at the beginning of the navigated surgery.

REFERENCE NUMBER LIST

| 1 | Operating Microscope |
| 5 | Microscope Tracker |
| 10 | Navigation Camera |
| 15 | Hybrid Tracker |
| 16 | Optical Pointer Instrument |
| 20 | Hybrid Navigation Unit |
| 30 | Pointer Instrument |
| 31 | EM Pointer Instrument |
| 40 | Patient Tracker |
| 50 | Field Generator |
| 90 | Operating Table |
| 100 | Patient |

**Claims**

1. A system for navigating surgical instruments (1) relative to a patient, comprising the following components:

   • a first tracking system for measuring the position of at least one of a first type of tracker;
   • a second tracking system for measuring the position of at least one of a second type of tracker;
   • at least one tracker (40) belonging to the first type of tracker for measuring the position of the patient (100);
   • a tracker (5) belonging to the second type of tracker for measuring the position of a surgical instrument (1) having at least one geometric primitive of interest; and
   • a connector (15) for rigidly connecting the at least one of the first type of tracker (40) with at least one of the second type of tracker (5) for registration and/or transformation of the coordinates of the surgical instrument (1) to the coordinates of the image data of the patient (100) and vice versa,

   wherein the at least two trackers (40, 5) of the first and second type are rigidly, but removably connected using the connector (15) allowing coupling of the separate trackers before or during surgery, wherein, when connected using the connector (15), the trackers (40, 5) are fixed relative to each other in a known or approximately known spatial relationship.

2. The system of claim 1, wherein the first tracking system is an electromagnetic tracking system and/or the second tracking system is an optical tracking system.

3. The system of claim 1 or 2, comprising a hybrid navigation unit (20) comprising at least a computer system for providing simultaneous support for the first and second tracking system, wherein, optionally, the at least one of a first type of tracker or the at least one of a second type of tracker is connected to the hybrid navigation unit (20).

4. The system of any one of claims 1 to 3, wherein the connector is a hybrid tracker (15) comprising stored information of pre-operatively known transformation between the trackers or wherein the poses of the trackers (40, 5) relative to each other are known from construction data.

5. The system of any one of claims 1 to 4, wherein the surgical instrument is electrically powered and its geometric primitive of interest is the tip of the surgical instrument.

6. The system of any one of claims 1 to 4, wherein the surgical instrument is an operating microscope (1) and the geometric primitives of interest are at least one of the image rectangle in the focal plane, the focus point or the camera origin of the operating microscope (1).

7. The system of claim 6, comprising a monitor for displaying the real microscopic image superimposed with a virtual microscopic image or an image injection module in the microscope (1) for displaying a virtual microscopic Image for visual perception of the surgeon when looking through the oculars.

8. A method for registering a surgical instrument relative to image data of a patient, the method comprising the steps of

 • connecting at least one of a first type of tracker (40) with at least one of a second type of tracker (5) by using a connector (15);
 • with a first tracking system, measuring the position of at least one of the first type of tracker (40) and measuring the position of the patient (100) by means of at least one tracker belonging to the first type of tracker (40);
 • with a second tracking system, measuring the position of at least one of the second type of tracker (5) and measuring the position of a surgical instrument (1) by using at least one of the second type of tracker (5); and
 • registering and/or transforming the coordinates of the surgical instrument (1) and its geometric primitive of interest to the coordinates of the image data of the patient (100) and vice versa,

 wherein the trackers (40, 5) are rigidly, but removably connected using the connector (15) pre- or intra-operatively by a user so that, when the trackers (40, 5) are connected, the trackers (40, 5) are rigidly fixed to each other in a known or approximately known spatial relationship.

9. The method of claim 8, using an electromagnetic tracking system as first tracking system and/or using an optical tracking system as second tracking system.

10. The method of any one of claims 8 to 9, comprising the step of using a device comprising stored information of pre-operatively known transformation between the trackers for registering and/or transforming the coordinates of a surgical instrument (1) and its geometric primitive of interest to the coordinates of the image data of the patient (100) and vice versa.

11. The method of any one of claims 8 to 10, comprising the step of connecting two or more trackers of both types of trackers, which are rigidly, but removably connected relative to each other, and the step of optimizing and/or verifying the transformation between these trackers.

12. The method of claim 11, wherein the at least two trackers are patient trackers and the transformation between the patient trackers is optimized and/or verified by performing at least one of the following steps:

 • two patient image registration procedures, one for the patient trackers of the at least one of a first type of tracker und one for the patient trackers of the at least one of a second type of tracker;
 • one patient image registration procedure using a navigated instrument equipped with trackers of both types of tracker;
 • sampling features whose spatial positions are known relative to a tracker of one type of tracker using a navigated instrument equipped with a tracker of the other type of tracker.

13. The method of any one of claims 8 to 12, using an electrically powered tool as surgical instrument, or using an operating microscope (1) as surgical instrument.

14. The method of claim 13, when using an operating microscope (1) as surgical instrument, wherein the coordinates of the image data of the patient (100) are transformed into the reference coordinate system of the microscopic camera enabling the generation of a virtual microscopic image.

15. The method of claim 14, wherein the virtual microscopic image is superimposed on the real microscopic image on a monitor of the navigation system, or wherein the virtual microscopic image is injected into the microscope beam path for visual perception of the surgeon when looking through oculars.


**Patentansprüche**

1. System zum Navigieren chirurgischer Instrumente (1) in Bezug auf einen Patienten, umfassend die folgenden Komponenten:

 • ein erstes Nachverfolgungssystem zum Messen der Position mindestens eines Trackers eines ersten Typs;
 • ein zweites Nachverfolgungssystem zum Messen der Position mindestens eines Trackers eines zweiten Typs;
 • mindestens einen Tracker (40) vom ersten Trackertyp zum Messen der Position des Patienten (100);
 • einen Tracker (5) vom zweiten Trackertyp zum Messen der Position eines chirurgischen Instruments (1) mit mindestens einem geometrischen Merkmal von Interesse; und
• einen Verbinder (15) zum festen Verbinden des mindestens einen Trackers (40) vom ersten Typ mit mindestens einem Tracker (5) vom zweiten Typ zum Registrieren und/oder Transformieren von Koordinaten des chirurgischen Instruments (1) mit den/in die Koordinaten der Bilddaten des Patienten (100) und umgekehrt,

wobei die mindestens zwei Tracker (40, 5) des ersten und des zweiten Typs fest aber lösbar unter Verwendung des Verbinders (15) verbunden sind, wodurch eine Verbindung der separaten Tracker vor oder während eines Eingriffs möglich ist, und wobei die Tracker (40, 5) relativ zueinander in einer bekannten oder näherungsweise bekannten räumlichen Beziehung fixiert sind, wenn diese mittels des Verbinders (15) verbunden sind.

2. System nach Anspruch 1, wobei das erste Nachverfolgungssystem ein elektromagnetisches Nachverfolgungssystem und/oder das zweite Nachverfolgungssystem ein optisches Nachverfolgungssystem ist.

3. System nach Anspruch 1 oder 2, umfassend eine hybride Navigationseinheit (20) umfassend mindestens ein Computersystem zum Bereitstellen einer gleichzeitigen Unterstützung für das erste sowie das zweite Nachverfolgungssystem, wobei der mindestens eine Tracker vom ersten Typ oder der mindestens eine Tracker vom zweiten Typ optional mit der hybriden Navigationseinheit (20) verbunden ist.

4. System nach einem der Ansprüche 1 bis 3, wobei der Verbinder ein hybrider Tracker (15) ist, der gespeicherte Informationen einer präoperativ bekannten Transformation zwischen den Trackern umfasst, oder wobei die Posen der Tracker (40, 5) relativ zueinander aus Konstruktionsdaten bekannt sind.

5. System nach einem der Ansprüche 1 bis 4, wobei das chirurgische Instrument elektrisch betrieben und dessen geometrisches Merkmal von Interesse die Spitze des chirurgischen Instruments ist.

6. System nach einem der Ansprüche 1 bis 4, wobei das chirurgische Instrument ein Operationsmikroskop (1) und das geometrische Merkmal von Interesse das Bildrechteck in der Brennebene, der Fokuspunkt und/oder der Kamera-Ursprung des Operationsmikroskops (1) ist.

7. System nach Anspruch 6, umfassend einen Monitor zum Darstellen des realen Mikroskopbildes in Überlagerung mit einem virtuellen Mikroskopbild oder ein Bildeinspeisungsmodul in dem Mikroskop (1) zum Darstellen eines virtuellen Mikroskopbildes zur visuellen Wahrnehmung des Chirurgen bei Blick durch die Okulare.

8. Verfahren zum Registrieren eines chirurgischen Instruments in Bezug auf Bilddaten eines Patienten, umfassend die Schritte

• Verbinden mindestens eines Trackers (40) eines ersten Typs mit mindestens einem Tracker (5) eines zweiten Typs unter Verwendung eines Verbinders (15);
• Messen der Position mindestens eines Trackers (40) vom ersten Typ und Messen der Position des Patienten (100) mittels mindestens eines Trackers (40) vom ersten Trackertyp mit einem ersten Nachverfolgungssystem;
• Messen der Position mindestens eines Trackers (5) vom zweiten Typ und Messen der Position eines chirurgischen Instruments (1) mittels mindestens eines Trackers (5) vom zweiten Typ mit einem zweiten Nachverfolgungssystem; und
• Registrieren und/oder Transformieren der Koordinaten des chirurgischen Instruments (1) und dessen geometrischen Merkmals von Interesse mit den/in die Koordination der Bilddaten des Patienten (100) und umgekehrt,

wobei die Tracker (40, 5) von einem Benutzer prä- oder intraoperativ fest aber lösbar mittels des Verbinders (15) derart verbunden werden, dass die Tracker (40, 5) untereinander in einer bekannten oder näherungsweise bekannten räumlichen Beziehung fest fixiert sind, wenn die Tracker (40, 5) verbunden sind.

9. Verfahren nach Anspruch 8, unter Verwendung eines elektromagnetischen Nachverfolgungssystems als erstes Nachverfolgungssystem und/oder eines optischen Nachverfolgungssystems als zweites Nachverfolgungssystem.

10. Verfahren nach Anspruch 8 oder 9, umfassend den Schritt des Verwendens einer Vorrichtung, die gespeicherte Informationen einer präoperativ bekannten Transformation zwischen den Trackern umfasst, zum Registrieren und/oder Transformieren der Koordinaten eines chirurgischen Instruments (1) und dessen geometrischen Merkmals von Interesse mit den/in die Koordinaten der Bilddaten des Patienten (100) und umgekehrt.

**11.** Verfahren nach einem der Ansprüche 8 bis 10, umfassend den Schritt des Verbindens zweier oder mehrerer Tracker beider Trackertypen, die fest aber lösbar miteinander verbunden sind, und den Schritt des Optimierens und/oder Verifizierens der Transformation zwischen diesen Trackern.

**12.** Verfahren nach Anspruch 11, wobei die mindestens zwei Tracker Patiententracker sind und die Transformation zwischen den Patiententrackern optimiert und/oder verifiziert wird durch das Durchführen mindestens eines der folgenden Schritte:

• zwei Patientenbildregistrierungsverfahren, eines für die Patiententracker des mindestens einen Trackers eines ersten Typs und eines für die Patiententracker des mindestens einen Trackers eines zweiten Typs;
• ein Patientenbildregistrierungsverfahren, das ein navigiertes Instrument verwendet, das mit Trackern beider Trackertypen ausgestattet ist;
• Probeentnahme von Merkmalen, deren räumliche Positionen relativ zu einem Tracker eines Trackertyps bekannt sind, mittels eines navigierten Instruments, das mit einem Tracker des anderen Trackertyps ausgestattet ist.

**13.** Verfahren nach einem der Ansprüche 8 bis 12, unter Verwendung eines elektrisch betriebenen Werkzeugs als chirurgisches Instrument oder unter Verwendung eines Operationsmikroskops (1) als chirurgisches Instrument.

**14.** Verfahren nach Anspruch 13, wobei, wenn ein Operationsmikroskop (1) als chirurgisches Instrument verwendet wird, die Koordinaten der Bilddaten des Patienten (100) in das Referenzkoordinatensystem der Mikroskopkamera, die das Erzeugen eines virtuellen Mikroskopbildes ermöglicht, transformiert werden.

**15.** Verfahren nach Anspruch 14, wobei das virtuelle Mikroskopbild auf einem Monitor des Navigaitonssystems dem realen Mikroskopbild überlagert wird oder wobei das virutelle Mikroskopbild zur visuellen Wahrnehmung durch den Chirurgen, der durch die Okulare blickt, in den Mikroskopstrahlpfad eingespeist wird.

## Revendications

**1.** Système de navigation d'instruments chirurgicaux (1) par rapport à un patient, comprenant les composants suivants :

• un premier système de suivi pour mesurer la position d'au moins un d'un premier type de dispositif de suivi ;
• un deuxième système de suivi pour mesurer la position d'au moins un d'un deuxième type de dispositif de suivi ;
• au moins un dispositif de suivi (40) appartenant au premier type de dispositif de suivi pour mesurer la position du patient (100) ;
• un dispositif de suivi (5) appartenant au deuxième type de dispositif de suivi pour mesurer la position d'un instrument chirurgical (1) ayant au moins une primitive géométrique d'intérêt ; et
• un connecteur (15) pour connecter de façon rigide l'au moins un du premier type de dispositif de suivi (40) avec au moins un du deuxième type de dispositif de suivi (5) pour enregistrement et/ou transformation des coordonnées de l'instrument chirurgical (1) en les coordonnées des données d'image du patient (100) et vice versa,

dans lequel les au moins deux dispositifs de suivi (40, 5) des premier et deuxième types sont connectés de façon rigide mais amovible en utilisant le connecteur (15) permettant le couplage des dispositifs de suivi séparés avant ou pendant une chirurgie, dans lequel, lorsqu'ils sont connectés en utilisant le connecteur (15), les dispositifs de suivi (40, 5) sont fixés l'un par rapport à l'autre dans une relation spatiale connue ou approximativement connue.

**2.** Système selon la revendication 1, dans lequel le premier système de suivi est un système de suivi électromagnétique et/ou le deuxième système de suivi est un système de suivi optique.

**3.** Système selon la revendication 1 ou 2, comprenant une unité de navigation hybride (20) comprenant au moins un système informatique pour fournir un support simultané pour les premier et deuxième systèmes de suivi, dans lequel, facultativement, l'au moins un d'un premier type de dispositif de suivi ou l'au moins un d'un deuxième type de dispositif de suivi est connecté à l'unité de navigation hybride (20).

**4.** Système selon l'une quelconque des revendications 1 à 3, dans lequel le connecteur est un dispositif de suivi hybride (15) comprenant des informations stockées de transformation connue en préopératoire entre les dispositifs de suivi ou dans lequel les poses des dispositifs de suivi (40, 5) l'un par rapport à l'autre sont connues à partir de données de construction.

5. Système selon l'une quelconque des revendications 1 à 4, dans lequel l'instrument chirurgical est électrique et sa primitive géométrique d'intérêt est la pointe de l'instrument chirurgical.

6. Système selon l'une quelconque des revendications 1 à 4, dans lequel l'instrument chirurgical est un microscope opératoire (1) et les primitives géométriques d'intérêt sont au moins un(e) parmi le rectangle d'image dans le plan focal, le foyer ou l'origine de caméra du microscope opératoire (1).

7. Système selon la revendication 6, comprenant un moniteur pour afficher l'image microscopique réelle superposée avec une image microscopique virtuelle ou un module d'injection d'image dans le microscope (1) pour afficher une image microscopique virtuelle pour une perception visuelle du chirurgien lorsqu'il regarde à travers les oculaires.

8. Procédé d'enregistrement d'un instrument chirurgical par rapport à des données d'image d'un patient, le procédé comprenant les étapes de

• connexion d'au moins un d'un premier type de dispositif de suivi (40) avec au moins un d'un deuxième type de dispositif de suivi (5) en utilisant un connecteur (15) ;
• avec un premier système de suivi, mesure de la position d'au moins un du premier type de dispositif de suivi (40) et mesure de la position du patient (100) au moyen d'au moins un dispositif de suivi appartenant au premier type de dispositif de suivi (40) ;
• avec un deuxième système de suivi, mesure de la position d'au moins un du deuxième type de dispositif de suivi (5) et mesure de la position d'un instrument chirurgical (1) en utilisant au moins un du deuxième type de dispositif de suivi (5) ;
et
• enregistrement et/ou transformation des coordonnées de l'instrument chirurgical (1) et de sa primitive géométrique d'intérêt en les coordonnées des données d'image du patient (100) et vice versa,

dans lequel les dispositifs de suivi (40, 5) sont connectés de façon rigide mais amovible en utilisant le connecteur (15) avant ou pendant une opération par un utilisateur de telle sorte que, lorsque les dispositifs de suivi (40, 5) sont connectés, les dispositifs de suivi (40, 5) sont fixés de façon rigide l'un à l'autre dans une relation spatiale connue ou approximativement connue.

9. Procédé selon la revendication 8, utilisant un système de suivi électromagnétique comme premier système de suivi et/ou utilisant un système de suivi optique comme deuxième système de suivi.

10. Procédé selon l'une quelconque des revendications 8 à 9, comprenant l'étape d'utilisation d'un dispositif comprenant des informations stockées de transformation connue en préopératoire entre les dispositifs de suivi pour enregistrement et/ou transformation des coordonnées d'un instrument chirurgical (1) et de sa primitive géométrique d'intérêt en les coordonnées des données d'image du patient (100) et vice versa.

11. Procédé selon l'une quelconque des revendications 8 à 10, comprenant l'étape de connexion de deux dispositifs de suivi ou plus des deux types de dispositifs de suivi, qui sont connectés de façon rigide mais amovible l'un par rapport à l'autre, et l'étape d'optimisation et/ou de vérification de la transformation entre ces dispositifs de suivi.

12. Procédé selon la revendication 11, dans lequel les au moins deux dispositifs de suivi sont des dispositifs de suivi de patient et la transformation entre les dispositifs de suivi de patient est optimisée et/ou vérifiée en mettant en œuvre au moins une des étapes suivantes :

• deux procédures d'enregistrement d'image de patient, une pour les dispositifs de suivi de patient de l'au moins un d'un premier type de dispositif de suivi et une pour les dispositifs de suivi de patient de l'au moins un d'un deuxième type de dispositif de suivi ;
• une procédure d'enregistrement d'image de patient utilisant un instrument en navigation équipé avec des dispositifs de suivi des deux types de dispositif de suivi ;
• échantillonnage de caractéristiques dont les positions spatiales sont connues par rapport à un dispositif de suivi d'un type de dispositif de suivi en utilisant un instrument en navigation équipé avec un dispositif de suivi de l'autre type de dispositif de suivi.

**13.** Procédé selon l'une quelconque des revendications 8 à 12, utilisant un outil électrique comme instrument chirurgical, ou utilisant un microscope opératoire (1) comme instrument chirurgical.

**14.** Procédé selon la revendication 13, lorsqu'utilisant un microscope opératoire (1) comme instrument chirurgical, dans lequel les coordonnées des données d'image du patient (100) sont transformées dans le système de coordonnées de référence de la caméra de microscope permettant la génération d'une image microscopique virtuelle.

**15.** Procédé selon la revendication 14, dans lequel l'image microscopique virtuelle est superposée sur l'image microscopique réelle sur un moniteur du système de navigation, ou dans lequel l'image microscopique virtuelle est injectée dans le trajet de faisceau de microscope pour une perception visuelle du chirurgien lorsqu'il regarde à travers les oculaires.

**Figure 1**

**Figure 2**

**Figure 3**

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2001034050 A2
- US 6235038 B1
- US 20120165657 A1
- US 20110054293 A1
- US 8494614 B2
- US 7702379 B2
- WO 2000039576 A1
- US 5831260 A

**Non-patent literature cited in the description**

- **EGGERS et al.** *Int. J. Oral and Max. Surg.,* 2006, vol. 35 (12), 1081-1095
- **MAINTZ ; VIERGEVER.** *Med. Int. Ana.,* 1998, vol. 2 (1), 1-36